# DEMANDE DE BREVET EUROPEEN

(11) **EP 1 369 117 A1**
(43) Date de publication de la demande: **10.12.2003**
(21) Numéro de dépôt: 02447114.6
(22) Date de dépôt: 07.06.2002
(51) Int. Cl.: A61K 31/5513, A61P 35/00

(54) **Utilisation de dérivés et de précurseurs d'oxaziridine dans le traitement de tumeurs cancereuses**

(71) Demandeur: UNIVERSITE LIBRE DE BRUXELLES, B-1050 Bruxelles (BE)
(72) Inventeur: Dubois, Jacques, 1495 Sart-Dames-Avelines (BE)
(74) Mandataire: Van Malderen, Michel

(57) **Abrégé**

La présente invention se rapporte à l'utilisation de dérivés et de leurs précurseurs (prodrogues) d'oxaziridine de formule I ou II pour la préparation d'un médicament destiné au traitement ou à la prévention des tumeurs cancéreuses.

## Description

### Objet de l'invention

La présente invention est relative à l'utilisation de dérivés et de précurseurs d'oxaziridine pour des applications topiques dans le cadre du traitement de tumeurs cancéreuses.

### Arrière-plan technologique à la base de l'invention

La chlorodiazépoxide (CDZ) est un produit utilisé depuis de nombreuses années en temps que tranquilisant chez l'humain.

Les métabolites majeurs du CDZ sont le desméthylchlorodiazépoxide (DES-CDZ) et le demoxepan (DEM), qui sont détectés en même temps que des métabolites mineurs tels que le desméthyldiazepam (DES-DIAZ) et l'oxazepam (OXAZ) après administration de chlorodiazépoxide (CDZ).

Soentjens-Werts et al. (Journal of Chromatography A, Vol. 662, p. 255-262, 1994 et Talanta, Vol. 42 n°4, p. 581-589, 1995) décrivent ces différents dérivés de chlorodiazépoxide obtenus suite à une irradiation par rayonnement UV. Ils suggèrent que la chlorodiazépoxide présente une caractéristique de phototoxicité et que ses métabolites présentent également une cytotoxicité.

### Buts de l'invention

La présente invention vise à fournir des produits et procédés permettant un traitement efficace, en particulier chez l'humain, de tumeurs cancéreuses. Un but particulier vise à traiter des tumeurs cancéreuses dans le cas d'une administration topique cutanée sur des muqueuses.

Un autre but de l'invention vise à fournir un produit et un procédé de traitement qui ne risquent pas d'occasionner d'effets secondaires et qui ne provoquent pas ou peu de brûlures ni de cicatrices suite au traitement sur les parties traitées.

### Eléments caractéristiques de l'invention

La présente invention est relative à l'utilisation de dérivés et de précurseurs (prodrogues) d'oxaziridine comprenant au moins un chaîne fonctionnelle de formule I ou II 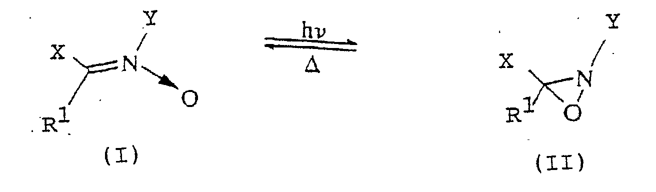 dans lesquelles X et Y représentent H ou un reste organique hydrocarboné linéaire incluant ou non des hétéroatomes, forment ensemble au moins un cycle organique incluant ou non des hétéroatomes utilisé pour la préparation d'un médicament dans le traitement ou la prévention des tumeurs cancéreuses.

Selon des formes d'exécution préférées de l'invention, ces dérivés ou précurseurs (prodrogues) d'oxaziridine comportent une chaîne soit de formule III ou IV, soit de formule V ou VI 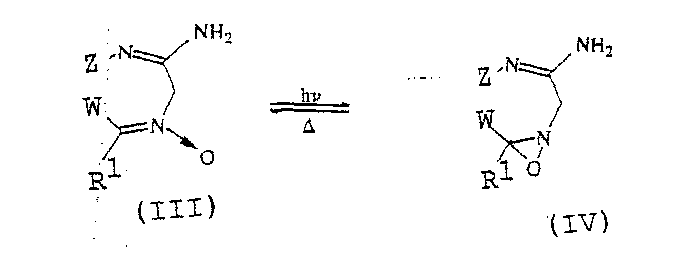 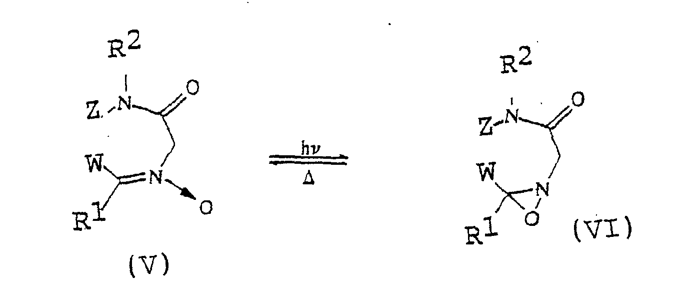 dans lesquelles W et Z forment ensemble un cycle à 6 atomes de carbone, de préférence le cycle de formule XI dans lequel R³ est un halogène, de préférence le chlore.

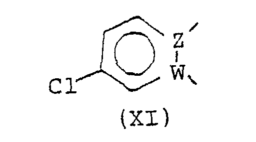

Dans lesdits dérivés et précurseurs (prodrogues) d'oxaziridine susmentionnés, R¹ représente H ou un groupement choisi parmi les groupes de type aryle ou alkyle et R² est H, un halogène ou un groupe alkyle, de préférence un groupement éthyle ou un groupement méthyle.

Les dérivés ou précurseurs (prodrogues) préférés de l'invention sont des précurseurs de chlorodiazépoxide tels que repris dans les formules VII à X 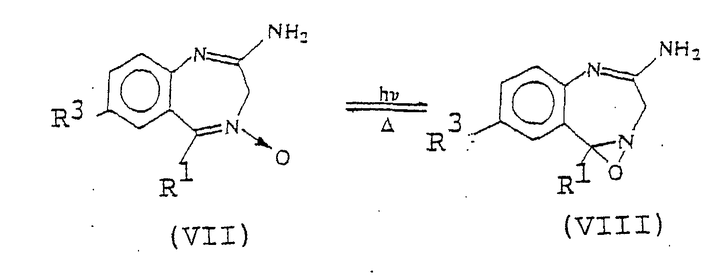 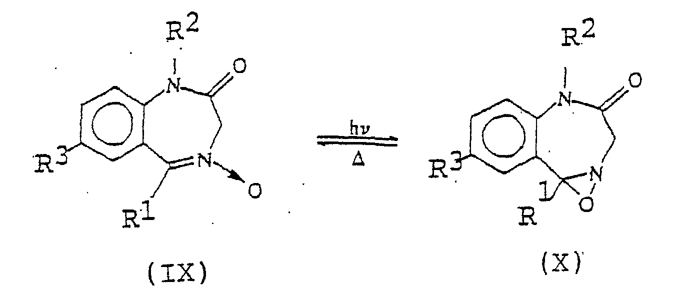 dans lesquels R¹, R² et R³ ont les définitions susmentionnées.

Lesdits composés, sont de préférence incorporés dans une composition pharmaceutique comprenant un véhicule ou diluant pharmaceutique adéquat et une quantité suffisante desdits dérivés ou de leurs précurseurs activés avantageusement par un ou plusieurs émission(s) de rayonnement UV, de préférence un rayonnement UV d'une longueur d'onde comprise entre 300 et 400 nanomètres (nm), plus particulièrement une longueur d'onde proche de 350 nanomètres (nm). Ladite composition pharmaceutique peut comprendre également un adjuvant adéquat tel qu'un produit antioxydant.

Le véhicule ou diluant pharmaceutique adéquat peut être choisi par l'homme de l'art en fonction du type d'administration et peut par exemple être sous forme solide, liquide, ou incorporé dans une composition gazeuse. Le rapport entre la quantité de véhicule ou diluant pharmaceutique adéquat et le composé actif varie en fonction du nombre de doses d'administration, de la surface de peau à traiter et des éventuels effets secondaires du produit sur le patient. De préférence, le véhicule pharmaceutique adéquat est un solvant adéquat pour solubiliser les composés de l'invention, de préférence une solution d'alcool, de préférence d'une concentration de l'ordre de 2% dans de l'éthanol.

Selon l'invention, lesdites tumeurs cancéreuses affectant des mammifères, en particulier l'être humain, traitées par les dérivés et précurseurs de l'invention ou ladite composition pharmaceutique sont des tumeurs malignes ou bénignes, des épithéliums ou muqueuses, telles que des tumeurs cancéreuses de la peau, du tube digestif telles que des tumeurs du colon, de l'intestin, de l'estomac, de l'oesophage, des tumeurs du poumon ou des tumeurs affectant les muqueuses des organes génitaux, en particulier des tumeurs du col de l'utérus.

Un autre aspect de l'invention est relatif à une trousse d'éléments (kit of parts) comprenant lesdits dérivés et/ou leurs précurseurs (prodrogues) ainsi qu'un moyen adéquat d'émission de rayonnement lumineux UV. De préférence, ledit rayonnement lumineux UV présente la majorité de son émission à une longueur d'onde comprise entre 300 et 400 nanomètres, de préférence à une longueur d'onde proche de 350 nanomètres et le moyen d'émission est adapté pour permettre que le rayonnement lumineux soit dirigé vers les dérivés et/ou leurs précurseurs situés sur la partie du corps du patient à traiter.

Cette trousse d'éléments comprend également un capillaire éventuellement incorporé à une ampoule comprenant lesdits dérivés et/ou leurs précurseurs pour permettre une administration topique aisée directement sur la partie du corps du patient à traiter. Lesdits composés de l'invention sont dissous dans une solution d'alcool de préférence d'une concentration de l'ordre de 2 % d'éthanol.

De préférence, l'ampoule comporte éventuellement diluée dans une solution adéquate une quantité suffisante desdits dérivés ou de leurs précurseurs pour effectuer un traitement complet de la tumeur cancéreuse.

De préférence, la composition pharmaceutique est sous forme liquide et le traitement vise à traiter la tumeur par un volume liquide comparable au volume de ladite tumeur.

Le traitement par les composés de l'invention peut consister également à imbiber une surface d'épithélium importante avec les composés de l'invention et à compléter le traitement par une irradiation complète de la surface de l'épithélium traitée.

Dans le cas d'un traitement nécessitant l'émission de rayonnement lumineux UV, de préférence à une longueur d'onde d'environ 350 nm, celui-ci est appliqué vers la partie du corps du patient à traiter pendant une durée suffisante mais avantageusement inférieure à 10 minutes, de préférence inférieure à 5 minutes, qui devrait suffire pour traiter ou éliminer complètement ladite tumeur. Cependant, en fonction de cas particuliers difficiles à traiter, il se peut que des séances répétitives de traitement soient effectuées pour traiter efficacement et éliminer complètement ladite tumeur.

De manière avantageuse, les composés de l'invention permettent que l'émission lumineuse puisse être arrêtée de manière à ce que les composés actifs n'agressent pas les tissus et cellules sains et évitent ainsi de provoquer des brûlures ou autres blessures douloureuses pour le patient sur les parties traitées (y compris les muqueuses).

Un dernier aspect de la présente invention est relatif à un procédé de traitement et/ou de prévention thérapeutique des tumeurs cancéreuses comprenant l'administration d'une quantité suffisante desdits dérivés ou de leurs précurseurs (éventuellement incorporés dans une composition pharmaceutique comprenant un véhicule pharmaceutique adéquat) sur une partie du corps d'un patient, comprenant lesdites tumeurs.

### Exemple :

Différents composés de l'invention ont été testés pour leurs activités anti-tumorales sur différentes lignées tumorales murines ou humaines et comparés à des produits anti-tumoraux connus.

Les lignées cellulaires testées sont la lignée lymphoblastoïde murine P388, la lignée de mélanome murin B16, la lignée de kératinocytes humains HaCaT, la lignée de mélanome humain HBL.

L'action anti-tumorale des composés de l'invention a été testée sur ces différentes lignées après différentes durées d'incubation (30 minutes, 2 heures, 3 heures, 48 heures ou 72 heures).

En outre, différents types d'irradiation, différentes durées d'irradiation (30 minutes ou 60 minutes) ainsi que différentes durées d'incubation ont été testés.

Les composés de l'invention testée sont le chlorodiazépoxide (CDZ), le demoxepan (DEM), le deschlorodiazépoxide, l'oxaziridine de chlorodiazépoxide, l'oxaziridine de demoxepan et l'oxaziridine de deschlorodiazépoxide.

Les différents composés de l'invention ont été comparés au Melphalan (TM).

Les différents résultats sont présentés dans le tableau 1 annexé.

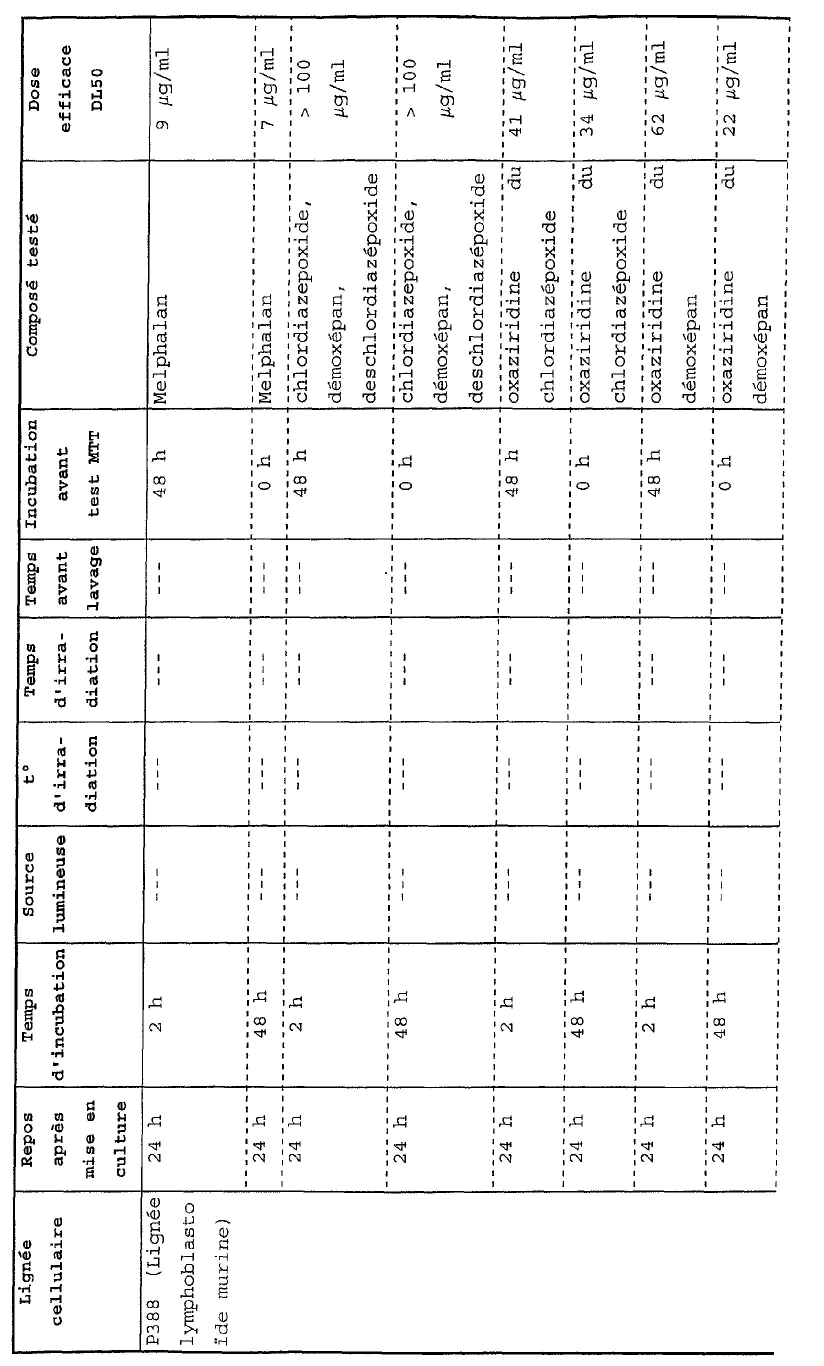

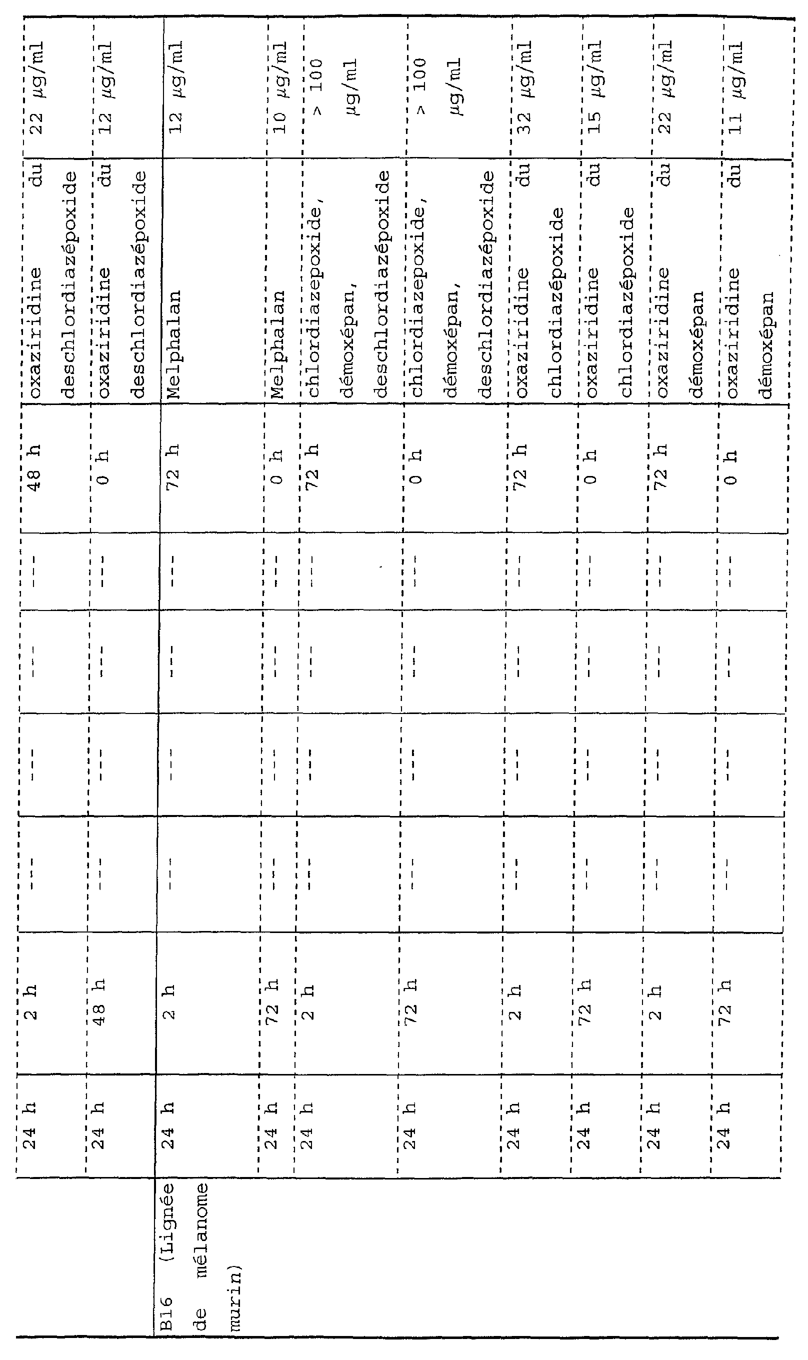

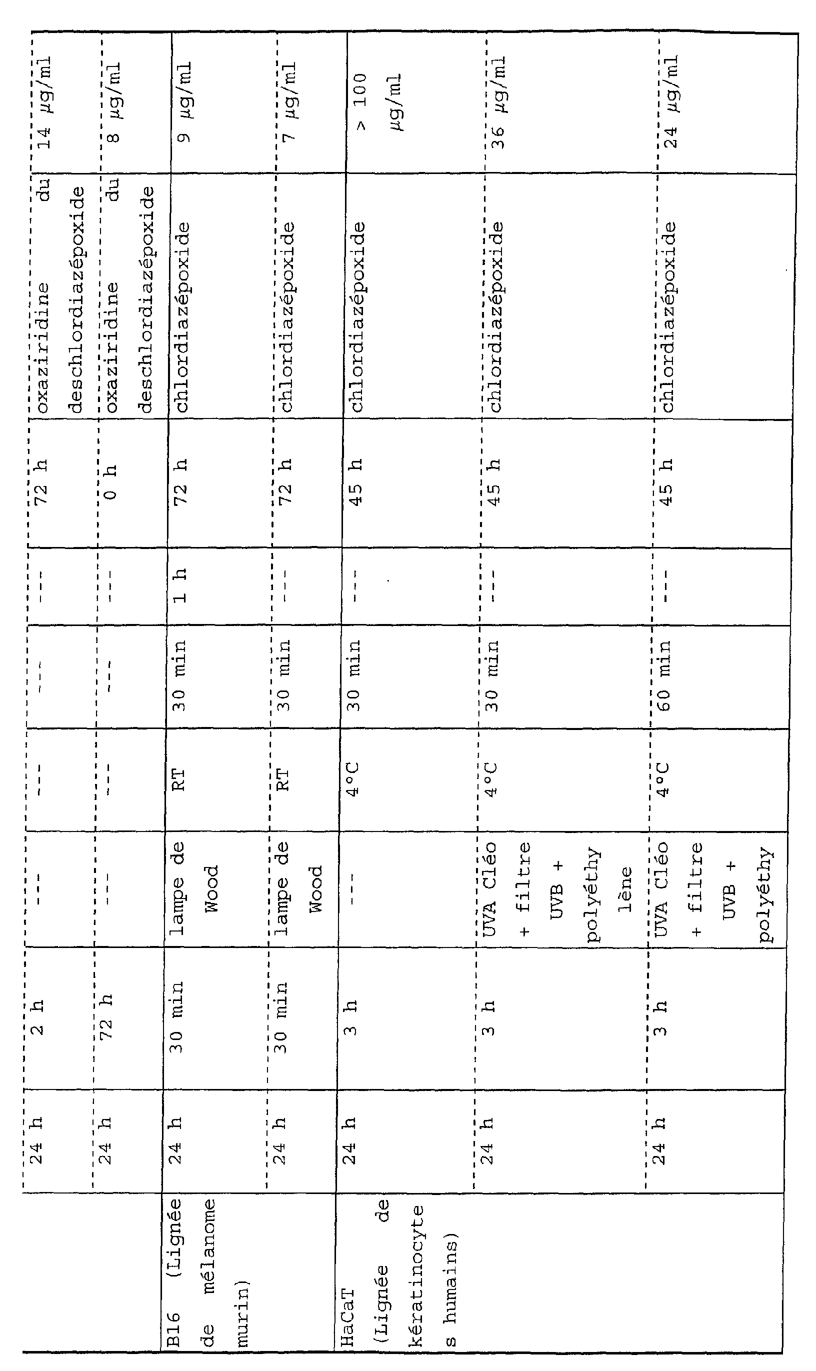

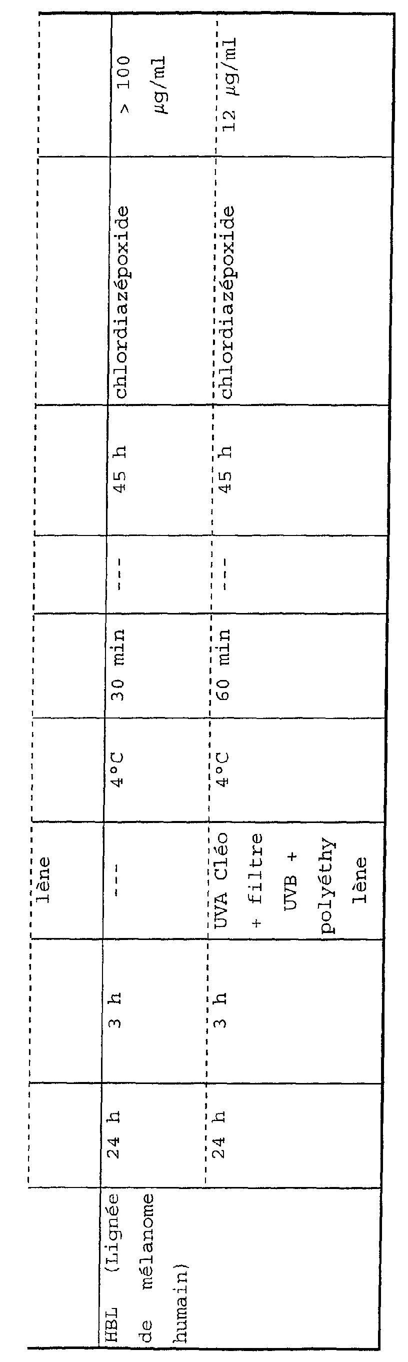

## Revendications

1. Utilisation de dérivés et de leurs précurseurs (prodrogues) d'oxaziridine comprenant au moins un chaînon fonctionnel de formule I ou II 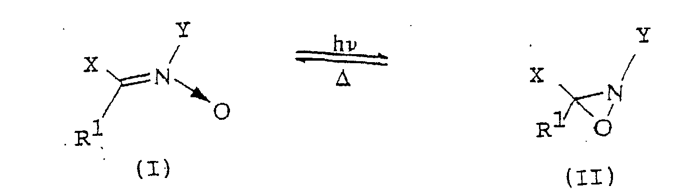 dans lesquels X et Y représentent H ou un reste organique hydrocarboné linéaire (incluant ou non des hétéroatomes) ou forment ensemble un cycle organique (incluant ou non des hétéroatomes) et dans lesquels R¹ représente H ou un groupement de type aryle ou alkyle, de préférence un cycle à 6 ou 7 atomes de carbone, dont un ou plusieurs carbones peuvent être éventuellement substitués par un groupement alkyle ou un halogène,
pour la préparation d'un médicament destiné au traitement ou à la prévention des tumeurs cancéreuses.

2. Utilisation selon la revendication 1, **caractérisée en ce que** les dérivés ou précurseurs d'oxaziridine comportent un chaînon fonctionnel de formule III ou IV 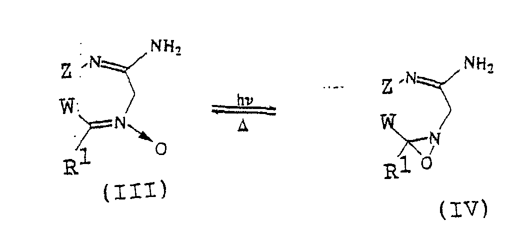 dans lesquelles W et Z forment ensemble un cycle à six atomes de carbone, de préférence un cycle de formule XI 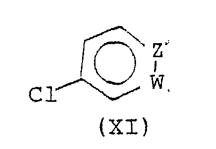 dans lesquels R3 représente un halogène, de préférence le chlore, et dans lesquels
R¹ est H ou un groupement de type aryle ou alkyle, de préférence un cycle de 6 ou 7 atomes de carbone, dont un ou plusieurs carbones peuvent être éventuellement substitués par un groupe alkyl ou un halogène, et
R² est H, un halogène ou un groupe alkyle, de préférence un groupe éthyle ou un groupe méthyle.

3. Utilisation selon la revendication 1 ou 2, **caractérisée en ce que** les dérivés ou précurseurs d'oxaziridine sont des précurseurs de chlorodiazépoxides tels que repris dans les formules VII et VIII 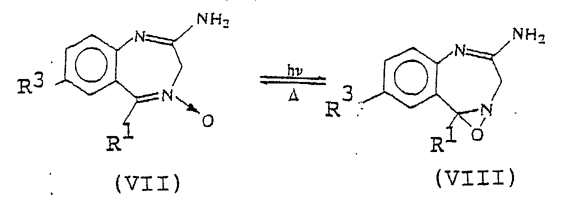 dans lesquelles R¹ est H ou un groupement aryle ou alkyle, de préférence un cycle à 6 ou 7 atomes de carbone dont un ou plusieurs carbone(s) peuvent éventuellement être substitués par un groupement alkyle ou un halogène,
R² est H, un halogène ou un groupement alkyle, de préférence un groupement éthyle ou un groupement méthyle,
R³ est un halogène, de préférence le chlore.

4. Utilisation selon la revendication 1, **caractérisée en ce que** les dérivés ou précurseurs d'oxaziridine comportent un chaînon fonctionnel de formule V ou VI 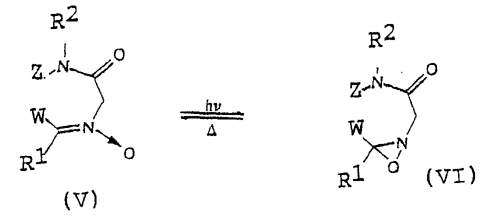 dans lesquels W et Z forment ensemble un cycle à 6 atomes de carbones, de préférence un cycle de formule XI 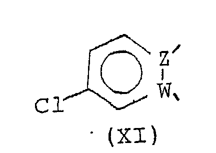 dans lesquels R³ représente un chlore, et dans lesquels R¹ est H ou un groupement aryle ou alkyle, de préférence un cycle à 6 ou 7 atomes de carbone, dont un ou plusieurs carbones peuvent être éventuellement substitués par un groupe alkyle ou un halogène.

5. Utilisation selon la revendication 1 ou 4, **caractérisée en ce que** les dérivés ou précurseurs d'oxaziridine sont des dérivés ou précurseurs de chlorodiazépoxides tels que repris dans les formules IX et X 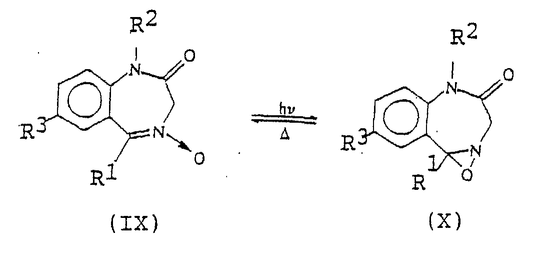 dans lesquelles R¹ est H ou un groupement aryle ou alkyle, de préférence un cycle à 6 ou 7 atomes de carbone, dont un ou plusieurs carbones peuvent être éventuellement substitués par un groupe aryle ou un halogène,
R2 est H ou un groupement alkyle, de préférence un groupement éthyle ou un groupement méthyle, et
R³ est un halogène, de préférence chlore.

6. Utilisation des dérivés ou de leurs précurseurs (prodrogue) d'oxaziridine selon l'une quelconque des revendications précédentes, **caractérisée en ce que** lesdites tumeurs cancéreuses des tumeurs malignes ou bénignes des épithéliums et muqueuses, telles que les tumeurs cancéreuses de la peau, les tumeurs cancéreuses du tube digestif, telles que les tumeurs du colon, de l'intestin, de l'estomac, de l'oesophage, les tumeurs affectant les organes génitaux, en particulier les tumeurs du col de l'utérus.

7. Trousse d'éléments comprenant des dérivés et précurseurs (prodrogues) d'oxaziridine tels que décrits dans l'une quelconque des revendications précédente, et un moyen d'émission de rayonnement lumineux UV adapté pour permettre l'émission de rayonnement lumineux UV dirigé vers lesdits dérivés et leurs précurseurs disposés sur les parties à traiter du corps d'un patient.

8. Trousse d'éléments selon la revendication 7 **caractérisée en ce que** le moyen d'émission du rayonnement lumineux UV a une longueur d'onde de 350 nm.
